# EUROPÄISCHE PATENTSCHRIFT

(11) **EP 0 598 778 B1**
(45) Veröffentlichungstag und Bekanntmachung des Hinweises auf die Patenterteilung: **14.11.2001**
(21) Anmeldenummer: 92916904.3
(22) Anmeldetag: 12.08.1992
(51) Int. Cl.: A61B 18/12

(54) **HOCHFREQUENZCHIRURGIEGENERATOR ZUM GEREGELTEN SCHNEIDEN UND KOAGULIEREN**
SURGICAL HIGH-FREQUENCY GENERATOR FOR CONTROLLED CUTTING AND COAGULATION
GENERATEUR DE HAUTE FREQUENCE UTILISE EN CHIRURGIE POUR COUPER ET COAGULER DE MANIERE REGLABLE

(30) Priorität: 12.08.1991 DE 4126609; 24.10.1991 DE 4135185
(43) Veröffentlichungstag der Anmeldung: 01.06.1994
(60) Teilanmeldung: 99122177.1
(73) Patentinhaber: Karl Storz GmbH & Co. KG, 78532 Tuttlingen (DE)
(72) Erfinder: LINDENMEIER, Heinz, D-8033 Planegg (DE); LOHR, Georg, D-8012 Ottobrunn (DE); FASTENMEIER, Karl, D-8000 München 83 (DE); FLACHENECKER, Gerhard, D-8012 Ottobrunn (DE)
(74) Vertreter: Münich, Wilhelm, Dr.
(86) Internationale Anmeldenummer: DE9200674
(87) Internationale Veröffentlichungsnummer: WO9303679

(56) Entgegenhaltungen:
- EP-A- 0 341 446
- DE-A- 3 420 340
- DE-A- 3 530 335
- DE-A- 3 622 337

## Beschreibung

### Technisches Gebiet

Die Erfindung bezieht sich auf einen Hochfrequenzgenerator für die Hochfrequenzchirurgie entsprechend dem Oberbegriff des Patentanspruchs 1.

### Stand der Technik

Hochfrequenzströme werden in der Chirurgie zum Abtragen von Gewebeteilen verwendet, wenn der Operationsort durch natürliche Körperöffnungen erreichbar ist, ein Skalpell aber nicht ohne Eröffnung des Körpers des Patienten angesetzt werden kann. Zum Beispiel können in der Urologie mit transurethral eingeführten Operationsinstrumenten und mit Hilfe von Hochfrequenzströmen Tumore aus der Blase abgetragen oder krankhafte Wucherungen der Prostata entfernt werden. In der Enterologie können auf ähnliche Weise z.B. Polypen von der Darmwand abgetrennt werden. Die Schneidelektrode des Operationsinstrumentes hat dabei nur solange eine Schneidwirkung, wie der den Hochfrequenzstrom liefernde Hochfrequenzgenerator aktiviert ist. Damit ist ein gefahrloses Einbringen und Entfernen des Operationsinstrumentes durch die natürlichen Körperöffnungen gewährleistet.

Weiterhin werden Hochfrequenzströme in der Chirurgie zum blutarmen Schneiden und zum Stillen von Blutungen verwendet. Es sind Hochfrequenzgeneratoren bekannt, die sowohl einen sogenannten "Schneidmodus" als auch einen "Koagulationsmodus" aufweisen. Diese Generatoren sind zum Gewebetrennen und zum gezielten Blutstillen, dem Koagulieren geeignet. Sie werden hauptsächlich bei endoskopischen Operationen wie z.B. in der Urologie, der Gynäkologie, der Polypektomie usw. angewandt. Daneben gibt es Hochfrequenzgeneratoren, die nur einen Koagulationsmodus besitzen. Diese sog. Koagulatoren werden in der offenen Chirurgie verwendet, um angeschnittene, stark blutende Gefäße zu verschließen oder großflächige, diffuse Blutungen zu stillen.

Ein Problem in der Hochfrequenzchirurgie ist die richtige Dosierung der momentan applizierten Hochfrequenzleistung. Die für gute Schneidwirkung mindestens notwendige Hochfrequenzleistung kann sehr stark schwanken. Sie hängt von der Gewebebeschaffenheit, der Leitfähigkeit und dem Wassergehalt des Gewebes, der Elektrodenform und Elektrodengröße, der Schnitt-Tiefe und Schnittgeschwindigkeit und weiteren elektrischen Parametern ab, die alle im Laufe einer Operation gewissen, oft sehr abrupt auftretenden Änderungen unterworfen sind. Die übliche, aus der Erfahrung des Operateurs gewonnene Einstellung des Hochfrequenzgenerators führt daher im Mittel zu einer deutlich überhöhten Hochfrequenzleistung, deren Gefahren der Operateur seinen Patienten und sich selbst bewußt aussetzen muß. Um diese Gefahren so klein wie möglich halten zu können bzw. nahezu vollständig ausschließen zu können, müßte die momentane Ausgangsleistung des Hochfrequenzgenerators automatisch so geregelt sein, daß sie in jedem Zeitmoment dem absolut notwendigen Mindestmaß entspricht.

Eine Erhöhung der zugeführten Leistung hat eine höhere Koagulation des Gewebes an der Schnittfläche zur Folge. Damit läßt sich eine Blutstillung erreichen. Diese ist bei Operationen in Geweben mit ohnehin geringer Blutungsneigung nicht unbedingt notwendig. Auch bei Operationstechniken, bei denen große Gewebevolumen abgetragen werden wie z.B. in der Urologie ist eine Schnittflächenkoagulation nicht unbedingt bereits während des Schneidens nötig. Denn hier wird mit den nachfolgenden Schnitten weiter in tieferliegende Gewebeschichten geschnitten. Eine geringe, definierte Koagulation ist jedoch häufig erwünscht um überflüssige Blutungen zu vermeiden und das Operationsfeld übersichtlich zu halten. Bei anderen Operationsarten, wie bei der Polypektomie, bei denen die ganze Operation im Optimalfall aus einem einzigen Schnitt besteht, ist jedoch bereits während des Schnittes eine optimale Koagulation unabdingbar.

Beim Schneiden mit Hochfrequenzströmen wird ein kontinuierlicher Hochfrequenzstrom verwendet. Bei der Hochfrequenzkoagulation wird die Joule'sche Wärme des Hochfrequenzstromes zur Stillung von Blutungen verwendet. Dazu wird ein Hochfrequenzstrom von einer Koagulationssonde auf das Gewebestück übergeleitet, auf dessen Oberfläche sich die Blutung befindet. Die Blutung kann von einem angeschnittenen Gefäß - meistens einer Arterie - oder großflächig in Form einer diffusen Blutung von vielen kleinen aufgetrennten Mikrogefäßen ausgehen. Bei der Koagulation kann man zwischen zwei Verfahren unterscheiden. Die Niederspannungskoagulation verwendet meist einen kontinuierlichen Hochfrequenzstrom, der so niedrig gewählt ist, daß ein Schneideffekt der Sonde nicht auftreten kann. Zur Hochspannungskoagulation dagegen verwendet man ausschließlich gepulste Hochfrequenzströme. Hier ist die mittlere Leistungszufuhr so gering, daß ein Schneideffekt der Sonde nicht auftreten kann. Andererseits sind die Spannungen so hoch, daß eine isoloierende Schicht an der Sonde, die durch Verschmutzung entstehen kann, durch einen Funken durchschlagen wird. Die Koagulation mit Spannungspulsen kann somit auch durchgeführt werden, wenn nicht sichergestellt werden kann, daß die Sonde beim Koagulieren metallisch blank ist.

Eine Vorrichtung zur Minimierung der Leistung beim Schneiden menschlichen Gewebes mit Hochfrequenzstrom ist in der Deutschen Patentschrift P 25 04 280 beschrieben.
Bei dieser Vorrichtung wird mit Hilfe einer Anzeigevorrichtung das Ausmaß des Lichtbogens zwischen der Schneidelektrode und dem zu schneidenden Gewebe festgestellt und das daraus abgeleitete elektrische Signal einer Regeleinrichtung zugeführt. Die Regeleinrichtung vergleicht dieses Signal mit dem Sollwertprogramm eines Sollwertgebers und leitet daraus eine Regelgröße ab, die die Ausgangsstromstärke des Generators so einstellt, daß die Intensität des Lichtbogens dem Sollwertprogramm folgt. Es hat sich aber gezeigt, daß mit konstanter Lichtbogenintensität nicht gleichzeitig ein konstantes Ergebnis der Koagulation, d.h. der Blutstillung der Schnittflächen verbunden ist. Offenbar sind die Bedingungen für gleichmäßiges Schneiden und gutes Koagulieren so verschieden, daß nicht beide Vorgänge mit einer einzigen, gemeinsamen Maßnahme, wie einer konstanten Lichtbogenintensität, gleichzeitig optimal durchgeführt werden können. Aus diesem Grund ist in der Deutschen Patentschrift P 25 04 280 ein Sollwertgeber vorgesehen, der nach einem Sollwertprogramm die Lichtbogenintensität so regelt, daß sich Zeitintervalle, in denen eine zum Schneiden erforderliche Stromstärke und Zeitintervalle, in denen eine zum Koagulieren notwendige Stromstärke des Hochfrequenzgenerators eingestellt ist, abwechseln. Zur Aufstellung eines Sollwertprogramms ist aber eine genaue Kenntnis der während einer Operation zu erwartenden Parameter, wie Gewebeart, Flüssigkeitsgehalt, Durchsetzung des Gewebes mit Blutgefäßen, Schnittgeschwindigkeit, Schnitt-Tiefe usw. nötig. In der Praxis ist daher die Aufstellung eines solchen Sollwertprogramms oft nur unvollkommen, manchmal auch gar nicht möglich. Selbst bei geringen Abweichungen der wirklichen von den angenommenen Parametern kann das Sollwertprogramm so weit vom Optimum abweichen, daß dann weder der Schneideffekt noch der Koagulationseffekt wirklich optimal sind.

Ein solches Sollwertprogramm ist auch unflexibel, wenn bei einem früher geführten Schnitt ein sehr großes Blutgefäß eröffnet wurde, das beim eingestellten Sollwertprogramm nicht vollständig verschlossen wurde. In diesem Fall muß in einem nachfolgenden Vorgang eine reine Koagulation ohne Schneidwirkung durchgeführt werden. Dies ist mit dem beschriebenen Sollwertprogramm nicht möglich, da sich hier Zeitintervalle mit Koagulationswirkung und Zeitintervalle mit Schneidwirkung gegenseitig abwechseln. Nach dem Stand der Technik ist in diesem Fall nur Koagulation mit gepulstem, ungeregeltem Koagulationsstrom möglich. Auch dabei können die Fälle mit zu hoher oder zu niedriger Generatoreinstellung auftreten. Bei zu hoher Leistungseinstellung des Hochfrequenzgenerators tritt wieder ein Lichtbogen mit allen beschriebenen Nachteilen auf, und gleichzeitig kann sich natürlich auch wieder die in diesem Moment unerwünschte Schneidwirkung einstellen. Bei zu geringer Leistungseinstellung des Hochfrequenzgenerators reicht die Koagulationswirkung nicht aus und die Blutung kann nicht oder nur ungenügend zum Stillstand gebracht werden.

Zur Lösung dieses Problems ist in der Deutschen Patentschrift DE 35 15 622 ein Generator beschrieben, der mit Hilfe von Meßeinrichtungen, einer Regeleinrichtung und eines zusätzlichen Zeitgebers die Ausgangsspannung des Generators so verändert, daß sich drei Zeitabschnitte mit genau beschreibbaren Zuständen der Ausgangsspannung in laufender Folge wiederholen.

Wie Messungen im Labor gezeigt haben, ist mit diesem Generator in vielen Fällen ein ausreichendes Operationsergebnis erreichbar. Dies ist besonders dann der Fall, wenn konstante Gewebeverhältnisse vorherrschen und die Schneidelektrode mit einer konstanten Geschwindigkeit durch das Gewebe bewegt wird. Ein praktischer Einsatz eines solchen Verfahrens ist denkbar in der Urologie. Hier ist die Schneidschlinge mechanisch starr an den Betätigungshebel gekoppelt, so daß sich diese mit einer definierten, konstanten Geschwindigkeit bewegen läßt. Auch ist hier das Operationsfeld so übersichtlich und alle möglichen Operationsstellen sind gut erreichbar, so daß im Falle einer mißlungenen Koagulation die blutende Stelle nachkoaguliert werden kann.

Bei anderen Operationstechniken wie die Polypektomie, bei der der Operationsort nur sehr schwer zugänglich und das Operationsfeld unübersichtlich ist, kann eine Nachkoagulation kaum mehr durchgeführt werden. Zur Abtragung von Polypen werden vorzugsweise Drahtschlingen verwendet, die über ein langes Endoskop an den Operationsort geführt werden. Wegen des mechanischen Aufbaus dieser Instrumente ist eine Bewegung der Schneidschlinge mit definierter Geschwindigkeit nicht möglich. Auch die Zugkraft läßt sich aufgrund der Reibung im Instrument nur sehr grob dosieren. Untersuchungen im Labor haben mit dem oben genannten Generator und typischen Polypektomieschlingen ein sehr schlecht reproduzierbares Koagulatuionsverhalten gezeigt. So ist bei manchen Schnitten eine durchaus gute Kaogulation erreichbar, während bei anderen Schnitten das Gewebe in kürzester Zeit ohne Koagulation glatt durchtrennt wird. Dieser Mangel tritt vor allen dann auf, wenn der Schnitt durch Bereiche mit unterschiedlichem Gewebearten führt oder wenn die Schneidelektrode eine Kruste aus Blutkoagel und verklebten Gewebeteile angesetzt hat.

Bei Koagulationen kommt es häufig vor, daß die Sonde mit der Gewebeoberfläche verklebt. Hier haften Gewebereste und koagulierte Eiweiße fest an der Sondenoberfläche und verbinden diese mit dem koagulierten Gewebe. Versucht nun der Operateur mit größerem Kraftauf wand von der Koagulationsstelle abzureißen, so löst sich häufig ein Teil der koagulierten Gewebeoberfläche mit ab. Als Folge kann die soeben koagulierte Blutung wieder beginnen. Dadurch wird eine weitere Koagulation notwendig. Außerdem erschwert eine mit Geweberesten verunreinigte Sonde weitere Koagulationen und muß vor der nächsten Koagulation gereingt werden. Bei endoskopischen Operationstechniken wie in der Urologie ist dies mit einem hohen Aufwand verbunden, da zuvor das Operationsinstrument aus dem Körper entfernt werden muß.

Um ein Verkleben der Sonde mit dem Gewebe zu verhindern, werden Koagulatoren, die ein flüssiges oder gasförmiges Medium zur Energieübertragung verwenden eingesetzt. Diese bauen zwischen der Elektrode und dem Gewebe eine leitfähige Zwischenschicht auf. Es sind auch Geräte, die einen ionisierten Gasstrahl zur Koagulation verwenden, bekannt. Diese sind zum Beispiel beschrieben in: H.D. Reidenbach, Hochfrequenz- und Lasertechnik in der Medizin, Springer Verlag, 1983. Hierbei ist allerdings der gerätetechnische Aufwand zur Flüssigkeits- bzw. Gaseinspeisung an die Elektrodenspitze beträchtlich. Wie Untersuchungen im Labor gezeigt haben, ist mit diesen Vorrichtungen nur eine oberflächliche Koagulation möglich. Tiefenkoagulationen und moderne bipolare Koagulationstechniken sind mit diesen Vorrichtungen nicht durchführbar. Dazu sind Sonden notwendig, die unmittelbar auf der Gewebeoberfläche aufliegen. Eine Verwendung der gleichen Sonde zum Schneiden und Koagulieren ist nicht möglich.

### Darstellung der Erfindung

Der Erfindung liegt daher die Aufgabe zugrunde, einen Hochfrequenzgenerator für die Hochfrequenzchirurgie zu schaffen, der unter Anpassung an die Gegebenheiten des Operationsgebietes eine zügige Gewebetrennung mit der minimal notwendigen Leistung bei gleichzeitiger definierter Blutstillung gewährleistet und der eine Hochfrequenzkoagulation derart ermöglicht, dass nach der Koagulation die Sonde vom Gewebe entfernt werden kann, ohne dass dabei die Gefahr besteht, die koagulierte Schicht abzureißen.

Diese Aufgabe wird erfindungsgemäß mit der in Patentanspruch 1 offenbarten Maßnahme gelöst. Weiterbildungen der Erfindung sind in den Unteransprüchen gekennzeichnet.

Aus der DE 35 30 335 A1 ist ein Hochfrequenz - Chierurgiegerät zum Schneiden und /oder Koagulieren biologischer Gewebe bekannt, das mindestens einen Hochfrequenzoszillator aufweist, der durch voneinander unabhängige Zeitgeber so gesteuert wird, dass einstellbare Zeitintervalle zum Schneiden oder Koagulieren erzeugt werden. Dem effektiven Schneide - oder Koagulationsintervall folgen jeweils einstellbare Pausenintervalle und nach deren Ablauf automatisch weitere Schneide- und Koagulationsintervalle, solange ein Tastenschalter zum Schneiden oder Koagulieren betätigt wird. Die vorliegende Erfindung geht von dem in diesem Dokument dargestellten Stand der Technik aus. Dabei liegt der Erfindung darauf aufbauend die Aufgabe zugrunde, die von der Regeleinrichtung eingestellte momentane Ausgangsgröße noch genauer und im Operationsverlauf besser angepasst als beim Stand der Technik zu steuern.

Mit Hilfe einer Einrichtung zur Feststellung des Zustandes des Gewebes in der Nähe der Schneidelektrode wird ein Hochfrequenzgenerator mit Regeleinrichtungen zur Vorgabe der momentanen elektrischen Ausgangsgrößen von einem Sollwertgeber eingestellt. Dazu wird mit Hilfe eines Zeitgebers in aufeinanderfolgenden Zeitintervallen aus mindestens zwei verschiedenen Betriebsarten mit unterschiedlicher operativer Zielsetzung die geeignete Betriebsart selektiert. Der zeitliche Verlauf der elektrischen Ausgangsgrößen des Generators wird mit Hilfe eines eines elektronischen Speichers mit Auswerteelektronik, dem die Ausgangsgröße der Meßeinrichtung zugeführt ist, gesteuert. Dazu werten die elektronischen Sollwertgeber die Ausgangsgröße der Meßeinrichtung und das Ausgangssignal des Speichers aus. Diese Anordnung kann unter Berücksichtigung der aktuellen Verhältnisse am Operationsort und des bisherigen zeitlichen Verlaufes der Operation die momentan erforderliche Betriebsart mit den optimalen Betriebsparametern einstellen. Zur technischen Realisierung der Anordnung ist es günstig eine gemischte Analog/Digitaltechnik zu verwenden oder einen Teil der Steuerfunktionen mit Hilfe eines Mikroprozessors oder eines Steuerrechners durchzuführen.

Eine besonders vorteilhafte Ausführung ist, daß ein Entscheider zum Ende eines Zeitintervalles automatisch entscheidet, welche Betriebsart in dem folgenden Intervall gewählt werden soll. Die Entscheidung wird aufgrund der Ausgangsgröße des elektronischen Speichers mit Auswerteelektronik und der Ausgangsgröße der Meßeinrichtung gefällt.

Eine weitere vorteilhafte Ausführung ist, daß ein Abschaltglied vorhanden ist, das die Zeitdauer des folgenden Zeitintervalls anhand der Ausgangsgröße der Messeinrichtung festlegt. Damit kann nach dem Erreichen des im Zeitintervall gewünschten Operationszieles dieses Zeitintervall beendet werden.

Um während des Schnittes den Zustand des Gewebes in unmittelbarer Nähe der Schneidelektrode festzustellen, ist es zweckmäßig eine Messeinrichtung in unmittelbarer Nähe der Schneidelektrode anzubringen.

Alternativ können auch durch eine Auswertung der elektrischen Signale am Generatorausgang die entsprechenden Informationen gewonnen werden. Ebenso ist es möglich mit Hilfe eines Prüfsignalgenerators ein Prüfsignal zu erzeugen, das der Schneidelektrode zugeführt wird. Mit einer Prüfsignalmesseinrichtung lässt sich der Zustand des Gewebes in der Nähe der Schneidelektrode ermitteln. Dieses Prüfsignal besitzt im allgemeinen eine so geringe Leistung, dass durch das Prüfsignal selbst keinerlei thermische Effekte im Gewebe auftreten. Damit darf es auch eingeschaltet sein, wenn der Leistungsgenerator abgeschaltet ist.

Eine vorteilhafte Ausführungsform besteht darin, dass mindestens eine Betriebsart mit der operativen Zielsetzung "Schneiden " und mindestens eine Betriebsart mit der operativen Zielsetzung " Koagulieren " selektiert werden können. Bei einem typischen Schnitt in Muskelgewebe würde sich eine zeitliche Abfolge von schneidenden und koagulieren den Betriebsarten einstellen. Wird nun z.B. ein Blutgefäß durchtrennt, so kann der Betriebsartenselektor bei Bedarf hintereinander mehrere Zeitintervalle mit koagulierenden Betriebsarten einstellen.

Zur Koagulation selbst gibt es mehrere Verfahren. Eines, das Koagulationen in größere Gewebetiefen erlaubt, arbeitet mit Generatorspannungen, die so niedrig eingestellt sind, daß ein Schneiden noch nicht möglich ist. Eine solche Betriebsart wird im Folgenden als " Niederspannungskoagulation " bezeichnet. Die Koagulation der Gewebeoberfläche auch in Gewebe- oder Knochenspalten ist mit kurzen Hochspannungspulsen möglich. Diese Pulse werden so kurz gewählt, das auch hier ein Schneiden nicht möglich ist.

Oftmals ist ist es notwendig, das Gewebe abkühlen zu lassen, deshalb ist es vorteilhaft, wenn Zeitintervalle mit vernachlässig geringer Generatorleistung selektiert werden können. In solchen Zeiten wird auch das durch den Lichtbogen entstehende Plasma abgebaut. Eventuell vorhandene isolierende Dampfschichten werden ebenfalls abgebaut.

In einer besonders vorteilhaften Ausführung des Generators besitzt die Meßeinrichtung eine Einrichtung zur Feststellung der an der Schneidelektrode vorliegenden Gewebeimpedanz. Aus dem Wert der Gewebeimpedanz kann die Entscheidung abgeleitet werden, ob im nächsten Zeitintervall koaguliert oder geschnitten werden soll. Niederohmige Impedanzen zeigen, daß keine Dampfschicht zwischen Schneidelektrode und Gewebe besteht oder daß sogar Blut ausgetreten ist und eine leitfähige Verbindung von Schneidelektrode zu Gewebe schafft. In diesem Falle muß im nächsten Zeitintervall koaguliert werden. Ist die Impedanz hochohmig, so besteht eine Dampfschicht, das Gewebe ist koaguliert und im nächsten Zeitintervall kann geschnitten werden.

Zur Realisierung einer Hochfrequenzkoagulation, bei der gemäß der Aufgabe der Erfindung nach der Koagulation die Sonde gefahrlos vom Gewebe entfernt werden kann ohne dabei die koagulierte Schicht zu beschädigen, wird der Hochfrequenzgenerator erfindungsgemäß zeitlich nacheinander in unterschiedlichen Betriebsarten gefahren. Eine erste Betriebsart (a) besitzt koagulierende Wirkung, während eine zweite Betriebsart (b) schneidende Wirkung besitzt.
Mit der Steuereinheit werden die Betriebsarten des Generators eingestellt; nach der Aktivierung des Generators zunächst die erste Betriebsart (a) mit koagulierender Wirkung und anschließend die zweite Betriebsart (b) mit schneidender Wirkung.

Zur Koagulation in der ersten Betriebsart (a) mit koagulierender Wirkung können je nach Anwendungsfall unterschiedliche Verfahren angewandt werden. Eine Koagulation
in größere Gewebetiefen kann durch die Applikation von niedrigen Spannungen erreicht werden, da dadurch das Gewebe langsam bis in tiefere Zonen erwärmt werden kann. Zur Verschorfung der Gewebeoberfläche ist die Koagulation mit kurzen Spannungspulsen hoher Amplitude zweckmäßig. Durch die hohe Spannung bildet sich ein Lichtbogen zum Gewebe aus und erreicht auch Stellen, die keinen ohmschen Kontakt zur Koagulationselektrode besitzen. Durch die hohe zugeführte Energie wird die Gewebeoberfläche schnell koaguliert und damit hochohmig. Dadurch wird ein weiterer Stromfluß, weitere Energiezufuhr und eine Erwärmung tieferliegender Gewebeschichten verhindert. Um einen Schneideffekt durch den Lichtbogen zu verhindern, wird die Generatorspannung üblicherweise gepulst, so daß die mittlere Leistung so gering ist, daß ein Schneiden nicht möglich ist.

Zur Koagulation können monopolare oder bipolare Elektrodenanordnungen eingesetzt werden. Bei bestimmten Operationstechniken wie in der Urologie, wird mit dem gleichen Instrument geschnitten und koaguliert, daher ist eine monopolare Koagulation zweckmäßig. Bei offenen Operationen kann jedoch eine separate Koagulationselektrode verwendet werden. Hier ist in vielen Fällen eine bipolare Koagulation günstiger, da diese einen definierten Stromfluß und damit eine definierte Wärmeentwicklung und Koagulation zwischen den Elektroden zur Folge hat.

In der zweiten Betriebsart (b) mit schneidender Wirkung stehen entsprechend den Anforderungen unterschiedliche Verfahren zur Verfügung. Wichtig ist hierbei, daß sich der zum Schneiden notwendige Lichtbogen mit Sicherheit trotz der koagulierten Gewebeoberfläche ausbildet. Dies kann durch Anlegen einer ausreichend hohen Spannung gewährleistet werden. Ist diese Spannung allerdings zu hoch, so wird überflüssige Energie in dem Patienten eingespeist. Dies kann mit einer Vorrichtung, wie sie im Deutschen Patent 2504280 beschrieben ist, vermieden werden. Bei dieser Vorrichtung wird mit Hilfe einer Anzeigevorrichtung das Ausmaß des Lichtbogens zwischen der Schneidelektrode und dem zu schneidenden Gewebe festgestellt und das daraus abgeleitete elektrische Signal einer Regeleinrichtung zugeführt. Die Regeleinrichtung vergleicht dieses Signal mit dem Sollwertprogramm eines Sollwertgebers und leitet daraus eine Regelgröße ab, die die Ausgangsstromstärke des Generators so einstellt, daß die Intensität des Lichtbogens dem Sollwertprogramm folgt. So wird nur die zur Aufrechterhaltung des zum Schneiden notwendigen Lichtbogens benötigte Energie abgegeben.

Eine Steuereinheit schaltet den Generator nach seiner Aktivierung zunächst in eine erste Betriebsart (a) mit koagulierender Wirkung. Nach dem Ende der Koagulation, das vom Operateur bestimmt oder vom Generator automatisch festgelegt wird, schaltet die Steuereinheit den Generator kurzzeitig in die zweite Betriebsart (b) mit schneidender Wirkung. Damit werden Zellreste, die die Gewebeoberfläche mit der Elektrode verkleben, verdampft. Beim Schneiden bildet sich an der Elektrode ein Lichtbogen aus, der jeweils zu den der Elektrode am nächsten liegenden Zellen überspringt. Diese verdampfen und es bildet sich zwischen Elektrode und Gewebeoberfläche eine Dampfschicht. Damit ist die Elektrode vom Gewebe getrennt und läßt sich leicht entfernen.

Eine besonders vorteilhafte Ausführungsform besteht darin, daß ein Zeitgeber vorhanden ist, der eine voreingestellte Zeit für das Zeitintervall Schneiden vorgibt. Diese Zeit ist so bemessen, daß sie zur Ausbildung eines Lichtbogens zwischen der Elektrode und dem Gewebe ausreicht. Sie ist jedoch so kurz, daß die Elektrode nicht nennenswert in das Gewebe eindringen kann. Für die Anwendungen, bei denen immer gleichartiges Gewebe koaguliert wird, kann die notwendige Zeit experimentell ermittelt und fest eingestellt werden.

Für Anwendungen, bei denen unterschiedliche Gewebearten koaguliert werden reicht unter Umständen eine fest vorgegebene Zeit nicht aus, um in allen Fällen eine Ablösung der Schlinge vom Gewebe zu erreichen. Deshalb wird in einer weiteren Ausführungsform die Vorrichtung zum Koagulieren so erweitert, daß eine Meßeinrichtung mit Auswerteeinrichtung vorhanden ist. Diese mißt während der ersten Betriebsart (a) mit koagulierender Wirkung die elektrischen Parameter am Generatorausgang und bestimmt daraus die optimale Zeitdauer für das nachfolgende Intervall mit der zweiten Betriebsart (b) mit schneidender Wirkung. Weiterhin ist ein Zeitgeber vorhanden, der die zweite Betriebsart (b) nach einer von der Meßeinrichtung mit Auswerteeinrichtung vorgegebenen Zeit beendet. Mit dem Ausgangssignal dieser Meßeinrichtung mit Auswerteeinrichtung wird der Zeitgeber entsprechend eingestellt. Zur Messung der elektrischen Parameter am Operationsort können die Ausgangsgrößen des Generators selbst oder das Signal eines Hilfsgenerators zur Messung herangezogen werden. Beispielhaft kann während der ersten Betriebsart (a) die Gewebeimpedanz ermittelt werden und für die zweite Betriebsart (b) eine Zeitdauer vorgegeben werden, die proportional zu dieser Impedanz ist. Dadurch wird für stärker verschorfte und damit hochohmigere Gewebeoberflächen eine längere Schnittdauer und damit auch ein besseres Ablösen der Sonde ermöglicht.

Eine andere vorteilhafte Ausführung besteht darin, daß eine Auswerteschaltung vorhanden ist, die den Schnittbeginn mittelbar oder unmittelbar feststellt. In der zweiten Betriebsart (b) mit schneidender Wirkung gibt diese ein Signal an die Steuereinheit ab, so daß das Zeitintervall mit der zweiten Betriebsart (b) eine voreinstellbare Zeit nach der Feststellung des Schneidens beendet wird. Um die Sonde partiell vom Gewebe zu lösen genügt eine schneidende Wirkung für kurze Zeit. Dieses Schneiden sollte allerdings noch eine geringfügige Zeit weitergeführt werden, so daß die Sonde auf ihrer ganzen Länge sicher vom Gewebe getrennt wird. Diese Zeit muß aber so kurz gewählt werden, daß die Sonde nicht wesentlich in das Gewebe eindringen kann.

Eine weitere vorteilhafte Ausführung besteht darin, daß die Auswerteschaltung einen mechanischen Sensor enthält, der ein Eindringen der Sonde in das Gewebe feststellt. So kann durch einen Wegaufnehmer der Abstand der Sonde von dem umliegenden Gewebe ermittelt werden. Hierfür eignen sich besonders optische Sensoren, die eine berührungsfreie Wegmessung ermöglichen.

Unmittelbar nach dem Beginn des Schneidens ändert sich die Impedanz zwischen der Sonde und dem Gewebe. Wie Messungen im Labor gezeigt haben, ist im allgemeinen beim Schnittbeginn ein deutlicher Anstieg dieser Impedanz meßbar. Dieser tritt besonders beim Schneiden nach einer Niederspannungskoagulation auf. Ist dagegen nach einer Hochspannungskoagulation das Gewebe sehr hochohmig, dann kann sich beim Beginn des Schneidens die Impedanz verringern. Hier überbrückt der auftretende Lichtbogen nun die hochohmige Gewebeschicht. Daher besteht eine vorteilhafte Ausführungsform darin, daß die Auswerteschaltung eine Einrichtung zur Messung und Auswertung der Impedanz enthält. Darin wird das Meßsignal mit einem Sollwert verglichen um die Impedanzänderung zu erkennen. Anstelle des Meßsignales kann auch eine Kombination des Meßsignales mit einer oder mehreren seiner Ableitungen zur Auswertung herangezogen werden.

Kennzeichnend für das Schneiden ist ist der dabei auftretende Lichtbogen. Daher kann der Schnittbeginn aufgrund einer Auswertung der spektralen Verteilung des Generatorausgangssignals erkannt werden. Eine solche Vorrichtung ist bereits in der noch nicht veröffentlichten deutschen Patentanmeldung P 4126607 beschrieben. Daher besteht eine vorteilhafte Ausführungsform darin, daß die Auswerteschaltung eine Einrichtung zur Auswertung der spektralen Anteile am Generatorausgang besitzt. Zur Auswertung kann das Generatorsignal selbst oder auch das Signal eines Hilfsgenerators oder eine Kombination aus beiden Signalen herangezogen werden. Die Auswertung selbst erfolgt durch einen Vergleich der Amplituden der spektralen Anteile die vom Generator bzw. dem Lichtbogen erzeugt werden.

### Kurze Beschreibung der Zeichnungen

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird. Es zeigen:
Fig. 1 : Prinzipschaltbild eines erfindungsgemäßen Hochfrequenzchirurgiegenerators zum koagulierenden Schneiden,
Fig. 2 : Beispielhafte Darstellung der Generatorausgangsspannung im Verlauf von drei Zeitintervallen,
Fig. 3 : Beispielhafte Darstellung der Generatorausgangsspannung für einen koagulierenden Schnitt mit 4 unterschiedlichen operativen Zielsetzungen,
Fig. 4 : Prinzipschaltbild des Hochfrequenzchirurgiegenerators zur Ausführung der Hochfrequenzkoagulation.

### Beschreibung eines Ausführungsbeispiels

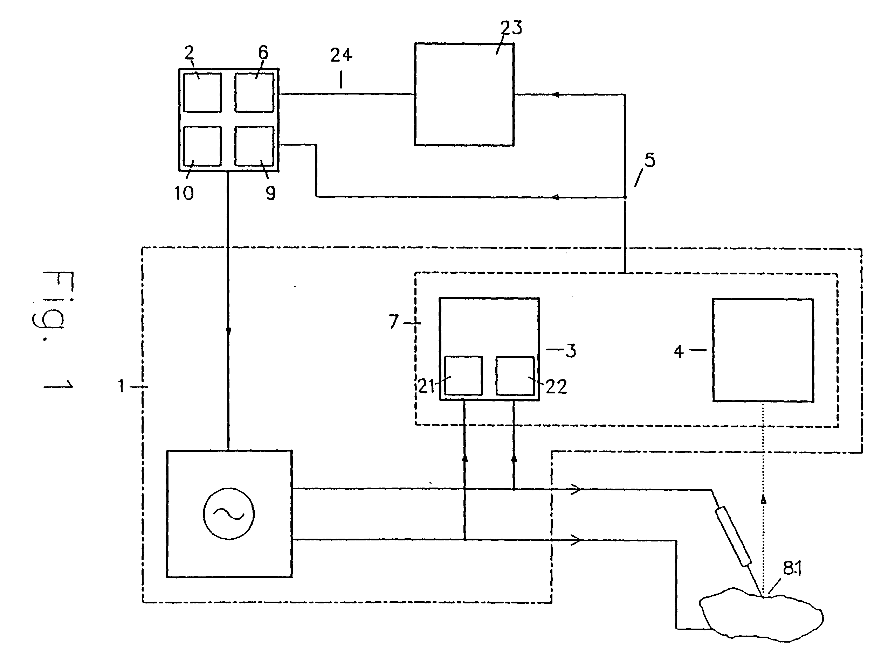

In Fig. 1 ist das Prinzipschaltbild des Hochfrequenzchirurgiegenerators nach der Erfindung dargestellt. Der Hochfrequenzgenerator (1) für die Hochfrequenzchirurgie besitzt Regeleinrichtungen zur Einstellung der momentanen elektrischen Ausgangsgrößen wie z.B. Strom, Spannung und Leistung und eine Einrichtung (7) zur unmittelbaren und / oder mittelbaren Feststellung des Zustands des Gewebes in der Nähe der Schneideelektrode (8.1).

Diese Einrichtung kann einen Meßgeber (4) in unmittelbarer Nähe der Schneidelektrode enthalten. Außerdem kann sie eine Auswerteeinrichtung zur Auswertung der elektrischen Ausgangsgrößen des Generators enthalten. Wahlweise kann die Einrichtung zur unmittelbaren und / oder mittelbaren Feststellung des Zustands des Gewebes in der Nähe der Schneideelektrode einen Prüfsignalgenerator (21) enthalten, dessen Prüfsignal der Schneidelektrode zugeführt und mit Hilfe der Prüfsignalmeßeinrichtung gemessen wird. Weiterhin enthält die Vorrichtung einen elektronischen Speicher mit Auswerteelektronik (23), dem die Ausgangsgröße (5) der Meßeinrichtung (7) zur unmittelbaren und / oder mittelbaren Feststellung des Zustands des Gewebes in der Nähe der Schneideelektrode zugeführt ist. Die Ausgangsgröße (24) des elektronischen Speichers mit Auswerteelektronik und die Ausgangsgröße (5) der Meßeinrichtung (7) werden dem Abschaltglied (2), dem Entscheider (6), dem Sollwertgeber (9) und dem Zeitintervallgeber (10) zugeführt.

Das Abschaltglied (2) begrenzt die Dauer des folgenden Zeitintervalles (13) anhand der Ausgangsgröße der Meßeinrichtung (7). Der Entscheider (6) wählt am Ende (15) des vorangegangenen Zeitintervalles (12) die Betriebsart für das folgende Zeitintervall (13) automatisch. Der elektronische Sollwertgeber (9) bestimmt den zeitlichen Verlauf der elektrischen Ausgangsgrößen des Hochfrequenzgenerators für die gewählte Betriebsart.
Ein Zeitintervallgeber (10) legt die Dauer eines jeden Zeitintervalles aufgrund der Ausgangsgrößen des elektronischen Speichers mit Auswerteelektronik fest.

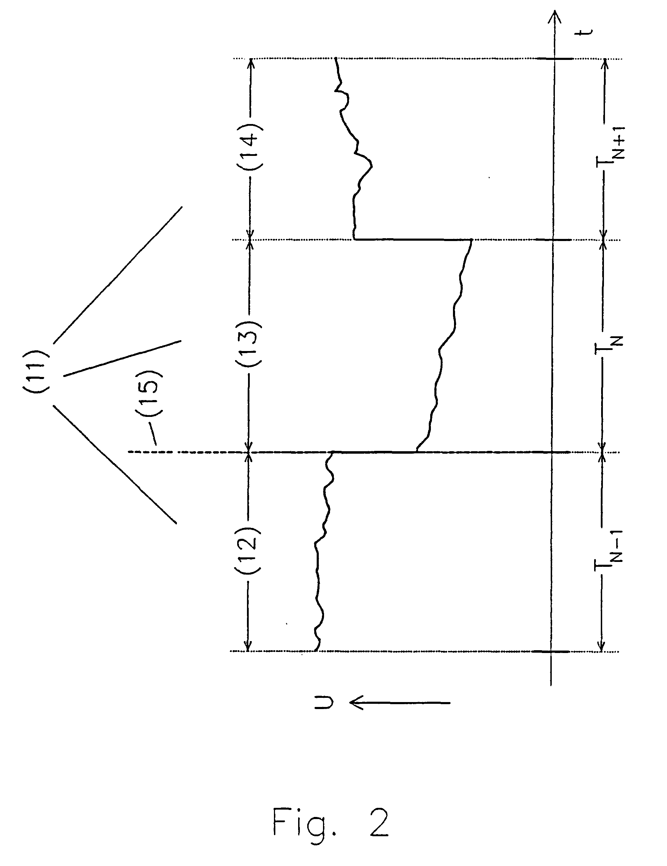

In Fig. 2 ist beispielhaft die Generatorausgangsspannung während dreier Zeitintervalle (11) dargestellt. Das folgende Zeitintervall (13) mit seiner Dauer T N , auf die sich jeweils die Erklärungen beziehen, wird begrenzt durch das vorhergehende Zeitintervall (12) mit seiner Dauer T _{N-1} und das übernächste Zeitintervall (14) mit der Dauer T _{N+1}.
Der Zeitpunkt (15) befindet sich an der Grenze zwischen dem vorhergehenden und dem momentanen Zeitintervall.

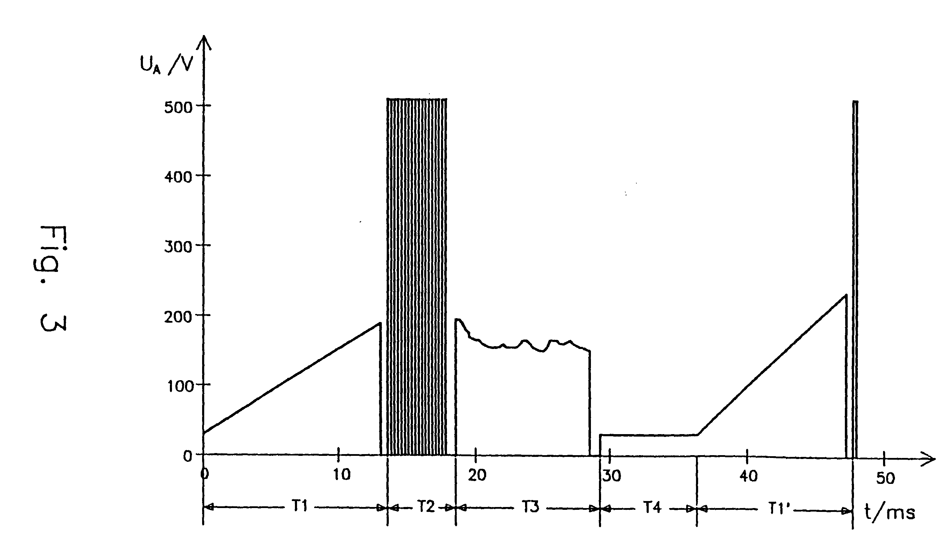

In Fig. 3 ist beispielhaft die Generatorausgangsspannung für eine typische Anwendung zur Polypektomie dargestellt, wo eine ausreichende Koagulation in jedem Falle sichergestellt werden muß. Im Zeitintervall T1 folgt die Generatorspannung einer ansteigenden Flanke. Die Generatorspannung ist verglichen mit den nachfolgenden Intervallen relativ niedrig. In dieser Zeit erfolgt ein Verkochen des Gewebes und damit eine Koagulation.
Die Stromverteilung im Gewebe in unmittelbarer Nähe der Schneidelektrode entspricht näherungsweise einem Zylinderfeld, mit dem sich die größte Tiefenwirkung erreichen läßt.
Das Zeitintervall T1 wird automatisch beendet, wenn die Einrichtung zur Feststellung des Zustandes des Gewebes das Auftreten eines Lichtbogens zwischen Schneidelektrode und Gewebe detektiert. Es folgt nun das Zeitintervall T2 in dem kurze Spannungsimpulse hoher Amplitude an das Gewebe abgegeben werden. Bei diesen Spannungsimpulsen tritt immer ein Lichtbogen auf. Die Pulse sind allerdings so kurz gewählt, daß der durch sie verursachte Schneideffekt vernachlässigbar ist. Damit erzielt man im Zeitintervall T2 eine Versiegelung der Oberfläche. Das Strömungsfeld des elektrischen Stromes im Gewebe ist vom Übertrittsort des Funkens ausgehend kugelsymmetrisch. Die Tiefenwirkung der Koagulation ist relativ gering. Nach einer voreingestellten Zeit ist dieses Koagulationsintervall beendet. Als nächstes folgt mit T3, ein Intervall in dem geschnitten wird. Bei dem dargestellten Beispiel erfolgt der Schnitt mit einer Regelung des Ausmaßes des beim Schneiden auftretenden Lichtbogens. Die Dauer des Intervalles T3 ist davon abhängig, wie lange das Intervall T1 gedauert hat. Messungen zeigten, daß bei langer Dauer des Intervalles T1 auch das Intervall T3 lange dauern darf, ohne daß weiter geschnitten wird, als koaguliert wurde. Anschließend folgt das Intervall T4, in dem der Generator nur geringe Ausgangsspannung abgibt. Diese geringe Spannung dient dazu, die Gewebeimpedanz zu messen. Solange die Gewebeimpedanz hochohmig ist besteht noch eine isolierende Dampfschicht zwischen Schneidelektrode und Gewebe. Das Gewebe muß nun solange abkühlen, bis diese Dampfschicht kondensiert ist und ein direkter ohmscher Kontakt zwischen Schneidelektrode und Gewebe besteht. Die Zeitdauer wird über die in der Meßeinrichtung (7) enthaltenen Einrichtung zur Feststellung der an der Schneidelektrode vorliegenden Gewebeimpedanz bestimmt. Anschließend folgt mit dem Intervall T1' wie der eine Phase zur Tiefenkoagulation mit einer rampenförmig ansteigenden Spannung. Messungen zeigten, daß man günstigste Schneid- und Koagulationseigenschaften erzielen kann, wenn während des Gewebetrennens alle Intervalle zur Tiefenkoagulation etwa gleich lange sind. Deshalb wird die Steilheit der Flanke entsprechend der Zeit gesteuert, die das letzte Intervall zur Tiefenkoagulation benötigte. War die Zeitdauer T1 länger als gewünscht, ist die Steigung der Rampe, wie gezeichnet steiler gewählt als im Intervall T1.

Bei zu kurzer Zeitdauer würde die Rampe der nächsten Intervalles zur Tiefenkoagulation entsprechend flacher eingestellt werden. Schnitte mit einem Ausgangssignal zeigten definierte Blutstillung. Unabhängig von der auf die Schneidelektrode ausgeübte Kraft ergab sich ein gleichmäßiger Schnitt.

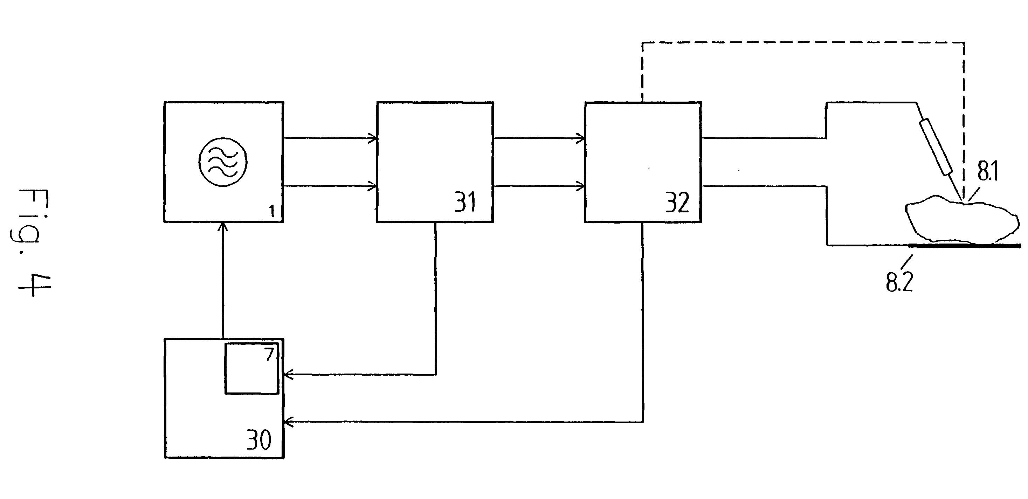

Fig. 4 zeigt das Prinzipschaltbild des Hochfrequenzchirurgiegenerators zur Ausführung der gefahrlosen Hochfrequenzkoagulation. Der Hochfrequenzgenerator (1) für die Hochfrequenzchirurgie mit einstellbarer Ausgangsleistung liefert einen hochfrequenten Strom über die Sonde (8.1) an das Gewebe. Der Strom fließt über eine weitere Elektrode (8.2) an den Generator zurück.
Die Elektrodenanordnung kann monopolar oder auch bipolar sein. Die monopolare Anordnung besteht aus einer kleinflächigen Sonde (8.1), die am Operationsort eingesetzt wird und einer großflächigen Elektrode (8.2), die an anderer Stelle am Körper des Patienten angebracht ist. Bei der bipolaren Anordnung besitzen die Sonde (8.1) und die Elektrode (8.2) gleich große Flächen und es werden beide am Operationsort mit gleicher Fläche appliziert. Mit Hilfe einer Steuereinheit (30) werden mindestens eine erste Betriebsart (a) mit koagulierender Wirkung und eine zweite Betriebsart (b) mit schneidender Wirkung realisiert. Ein Zeitgeber (10) in der Steuereinheit kann die Zeitdauer für die zweite Betriebsart (b) begrenzen. Die Meßeinrichtung (31) mit Auswerteeinrichtung führt während des Zeitintervalles mit der ersten Betriebsart (a) Messungen der elektrischen Parameter am Generatorausgang durch. Mit Hilfe der Meßergebnisse wird der Zeitgeber (10) für die zweite Betriebsart (b) voreingestellt. Eine Auswerteschaltung (32) dient zur mittelbaren und/oder unmittelbaren Feststellung des Schneidens. Sie gibt in der zweiten Betriebsart (b) ein Signal an die Steuereinheit (30) ab, wenn die Sonde (8.1) zu schneiden beginnt.

## Patentansprüche

1. Hochfrequenzchirurgiegenerator (1) zum geregelten Schneiden und Koagulieren, mit
- einem Hochfrequenzgenerator, dessen Ausgangssignal an eine Sonde angelegt ist,
- einer Regeleinrichtung zur Einstellung der momentanen elektrischen Ausgangsgrößen des Hochfrequenzgenerators, wie Strom, Spannung oder Leistung,
- einem elektronischen Sollwertgeber (9), dessen Ausgangssignal als Sollwert an die Regeleinrichtung zur Einstellung des zeitlichen Verlaufs der elektrischen Ausgangsgrößen des Hochfrequenzgenerators entsprechend der gewählten Betriebsart angelegt ist,
- einer Messeinrichtung (7) zur unmittelbaren und/oder mittelbaren Feststellung des Zustandes des Gewebes in der Nähe der Elektrode der Sonde (8.1), und einem Zeitintervallgeber (10),
**gekennzeichnet durch** folgende Merkmale:
- es ist elektronischer Speicher mit Auswerteelektronik vorgesehen, in dem das Ausgangssignal der Messeinrichtung gespeichert wird,
- am Ende jedes **durch** den Zeitintervallgeber vorgegebenen Zeitintervalls wertet der Sollwertgeber zur Einstellung des Sollwertes für die Regeleinrichtung das Ausgangssignal des Speichers mit Auswerteelektronik und das momentane Ausgangssignal der Messeinrichtung aus.

2. Hochfrequenzgenerator nach Anspruch 1,
**dadurch gekennzeichnet, dass** ein Entscheider (6) vorhanden ist, dem die Ausgangsgröße(5) der Messeinrichtung (7) zusammen mit der Ausgangsgröße (24) des elektronischen Speichers mit Auswerteeinrichtung (23) zugeführt ist und mit dessen Hilfe jeweils am Ende (15) des vorangegangenen Zeitintervalls (12) eine Entscheidung bezüglich der Wahl der Betriebsart für das folgende Zeitintervall (13) automatisch getroffen wird.

3. Hochfrequenzgenerator nach Anspruch 1 oder 2,
**dadurch gekennzeichnet, daß**
ein Abschaltglied (2) vorhanden ist, mit dessen Hilfe die Zeitdauer des folgenden Zeitintervalls (13) anhand der Ausgangsgröße der Einrichtung (7) zur unmittelbaren und / oder mittelbaren Feststellung des Zustands des Gewebes für die eingestellte Betriebsart eingestellt ist.

4. Hochfrequenzgenerator nach einem der Ansprüche 1 bis 3,
**dadurch gekennzeichnet, daß**
die Einrichtung (7) zur unmittelbaren und / oder mittelbaren Feststellung des Zustands des Gewebes in der Nähe der Schneidelektrode einen Meßgeber (4) in unmittelbarer Nähe der Schneidelektrode (8.1) enthält.

5. Hochfrequenzgenerator nach einem der Ansprüche 1 bis 3,
**dadurch gekennzeichnet, daß**
die Einrichtung (7) zur unmittelbaren und / oder mittelbaren Feststellung des Zustandes des Gewebes eine Meßeinrichtung zur Feststellung und eine Auswerteeinrichtung (3) zur Auswertung der elektrischen Ausgangsgrößen des Generators aufweist und daraus ein geeignetes Signal zur mittelbaren Feststellung des Zustandes des Gewebes gebildet ist.

6. Hochfrequenzgenerator nach einem der Ansprüche 1 bis 3,
**dadurch gekennzeichnet, daß**
die Einrichtung (7) zur unmittelbaren Feststellung des Zustands des Gewebes einen elektrischen Prüfsignalgenerator (21), dessen Prüfsignal der Schneideelektrode zugeführt ist, enthält und eine Prüfsignalmeßeinrichtung (22) zur Feststellung des Zustands des Gewebes in der Nähe der Schneideelektrode vorhanden ist.

7. Hochfrequenzgenerator nach einem der Ansprüche 1 bis 6,
**dadurch gekennzeichnet, daß**
grundsätzlich verschiedene Betriebsarten mit unterschiedlicher operativer Zielsetzung vorhanden sind, von denen eine Betriebsart " Schneiden " und die andere die Betriebsart " Koagulation " betrifft und gegebenenfalls weitere Betriebsarten für unterschiedliche Arten des Schneidens sowie unterschiedliche Arten des Koagulierens wählbar sind.

8. Hochfrequenzgenerator nach Anspruch 7,
**dadurch gekennzeichnet, daß**
während einer Betriebsart in den entsprechenden Zeitintervallen aus operativen Gründen die Ausgangsleistung des Generators vernachlässigbar klein gewählt ist.

9. Hochfrequenzgenerator nach einem der Ansprüche 1 bis 8,
**dadurch gekennzeichnet, daß**
in der Meßeinrichtung (7) eine Einrichtung zur Feststellung der an der Schneideelektrode (8.1) vorliegenden Gewebe impedanz vorhanden ist, und diese Impedanz anhand der Prüfsignale bzw. der elektrischen Ausgangsgrößen des Generators ermittelt wird
und die Betriebsart am Ende (15) des vorangegangenen Zeitintervalles (12) für das folgende Zeitintervall (13) auf Koagulation eingestellt wird, wenn die Lastimpedanz niedriger ist als ein vorgegebener Sollwert und die Betriebsart Schneiden eingestellt
wird, wenn die Lastimpedanz diesen Sollwert überschreitet.

10. Hochfrequenzgenerator nach einem der Ansprüche 7 bis 9,
**dadurch gekennzeichnet, daß**
mindestens vier verschiedene Betriebsarten mit unterschiedlicher operativer Zielsetzung vorhanden sind, von denen eine Betriebsart " Schneiden " und zwei andere die Betriebsarten " Koagulation " betreffen.

11. Hochfrequenzgenerator nach einem der Ansprüche 1 bis 10,
**dadurch gekennzeichnet, daß**
in der Betriebsart " Schneiden " die Ausgangsspannung des Hochfrequenzgenerators (1) auf einen vorgegebenen konstanten Wert geregelt wird.

12. Hochfrequenzgenerator nach einem der Ansprüche 1 bis 10,
**dadurch gekennzeichnet, daß**
in der Betriebsart " Schneiden " der Ausgangsstrom des Hochfrequenzgenerators (1) auf einen vorgegebenen konstanten Wert geregelt wird.

13. Hochfrequenzgenerator nach einem der Ansprüche 1 bis 10,
**dadurch gekennzeichnet, daß**
in der Betriebsart " Schneiden " die Ausgangsleistung des Hochfrequenzgenerators (1) auf einen vorgegebenen konstanten Wert geregelt wird.

14. Hochfrequenzgenerator nach einem der Ansprüche 1 bis 10,
**dadurch gekennzeichnet, daß**
in der Betriebsart " Schneiden " der beim Schneiden an der Schneidelektrode (8.1) entstehende Lichtbogen auf einen vorgegebenen konstanten Wert geregelt wird.

15. Hochfrequenzgenerator nach einem der Ansprüche 1 bis 14,
**dadurch gekennzeichnet, daß**
in der Betriebsart " Koagulation " wahlweise mit einer konstanten oder zeitveränderlichen Spannung koaguliert wird, die so niedrig ist, das ein Lichtbogen an der Schneidelektrode (8.1) nicht auftreten kann.

16. Hochfrequenzgenerator nach einem der Ansprüche 1 bis 14,
**dadurch gekennzeichnet, daß**
in der Betriebsart " Koagulation " mit Pulspaketen einer Spannung koaguliert wird, die so hoch ist, das ein Lichtbogen an der Schneidelektrode (8.1) auftritt.

17. Hochfrequenzgenerator nach Anspruch 10 bis 14,
**dadurch gekennzeichnet, daß**
eine der beiden unterschiedlichen Betriebsarten einer Koagulation nach Anspruch 15 und die andere Betriebsart einer Koagulation nach Anspruch 16 entspricht.

18. Hochfrequenzgenerator nach Anspruch 7 bis 17,
**dadurch gekennzeichnet, daß**
in der Betriebsart " Schneiden " der Generator (1) so gesteuert wird daß der Schnitt um maximal einen Elektrodendurchmesser tiefer in das Gewebe eindringt, als die Tiefe der vorhergehenden Koagulationen beträgt.

19. Hochfrequenzgenerator nach Anspruch 18,
**dadurch gekennzeichnet, daß**
in der Betriebsart " Schneiden " der Schnitt beendet wird, wenn die Impedanz des Gewebes stark absinkt.

20. Hochfrequenzgenerator nach einem der Ansprüche 1 bis 19,
**dadurch gekennzeichnet, daß**
die Zeitintervalle (11) jeweils eine vorgegebene, konstante Zeitdauer besitzen und die momentane Ausgangsgröße des Generators (1) wie Spannung, Strom, Leistung aus der vorhergehenden Periode ermittelt wird.

21. Hochfrequenzgenerator nach einem der Ansprüche 1 bis 19,
**dadurch gekennzeichnet, daß**
die Ausgangsgröße des Generators (1) auf einen für jedes Intervall vorgegebenen Wert eingestellt ist und die Zeitdauer der Zeitintervalle (11) jeweils aus der vorhergehenden Periode ermittelt wird.

22. Hochfrequenzgenerator nach einem der Ansprüche 1 bis 19,
**dadurch gekennzeichnet, daß**
die Ausgangsgröße des Generators (1) auf einen vorgegebenen Wert eingestellt ist, der sich aus den Messungen im vorhergehenden Zeitintervall ergibt und die Zeitdauer der Zeitintervalle (11) jeweils aus der vorhergehenden Periode ermittelt wird.

23. Hochfrequenzgenerator nach Anspruch 22,
**dadurch gekennzeichnet, daß**
die Ausgangsspannung des Generators aus der Gewebeimpedanz im vorhergehenden Intervall ermittelt wird.

24. Hochfrequenzgenerator nach einem der Ansprüche 7 bis 23,
**dadurch gekennzeichnet, daß**
in einem Zeitintervall der Betriebsart " Koagulation " mindestens so tief koaguliert wird, wie in dem letzten vorhergehenden Zeitintervall der Betriebsart " Schneiden " geschnitten wurde.

25. Hochfrequenzgenerator nach einem der Ansprüche 7 bis 24,
**dadurch gekennzeichnet, daß**
das erste Zeitintervall der Betriebsart " Koagulation " nach einem Zeitintervall der Betriebsart " Schneiden " einer Koagulation nach Anspruch 15 entspricht.

26. Hochfrequenzgenerator nach einem der Ansprüche 7 bis 25,
**dadurch gekennzeichnet, daß**
zu Beginn eines Zeitintervalles der Betriebsart " Koagulation " aufgrund der Gewebeimpedanz entschieden wird, ob eine Koagulation nach Anspruch 15 oder eine Koagulation nach Anspruch 16 durchgeführt wird. Bei einer hohen Gewebeimpedanz wird die Koagulation nach Anspruch 16 gewählt, während bei einer niedrigen Gewebeimpedanz eine Koagulation nach Anspruch 15 gewählt wird.

27. Hochfrequenzgenerator nach einem der Ansprüche 7 bis 26,
**dadurch gekennzeichnet, daß**
in der Betriebsart " Koagulation " eine Koagulation nach Anspruch 15 beendet wird, sobald ein Lichtbogen an der Schneideelektrode (8.1) auftritt.

28. Hochfrequenzgenerator nach Anspruch 27,
**dadurch gekennzeichnet, daß**
die Ausgangsspannung des Generators nach einer vorgegebenen Funktion von einem vorgegebenen Startwert aus ansteigt bis ein Lichtbogen an der Schneideelektrode (8.1) auftritt.

29. Hochfrequenzgenerator nach Anspruch 28,
**dadurch gekennzeichnet, daß**
der Anstieg der Ausgangsspannung aus dem vorhergehenden Intervall so bestimmt wird, daß die Zeit bis zum Auftreten eines Lichtbogens annähernd einem voreingestellten Wert entspricht.

30. Hochfrequenzgenerator nach einem der Ansprüche 7 bis 27,
**dadurch gekennzeichnet, daß**
die Ausgangsspannung des Generators in der Betriebsart " Koagulation " aufgrund des Auftretens eines Lichtbogens im vorhergehenden Intervall selektiert wird.

31. Hochfrequenzgenerator nach einem der Ansprüche 7 bis 27,
**dadurch gekennzeichnet, daß**
die Ausgangsspannung des Generators in der Betriebsart " Koagulation " erhöht wird, wenn nach einer vorbestimmten Zeitdauer noch kein Lichtbogens aufgetreten ist.

32. Hochfrequenzgenerator nach Anspruch 27,
**dadurch gekennzeichnet, daß**
ein zusätzliches Zeitintervall mit vernachlässigbar geringer Generatorleistung und ein weiteres Zeitintervall mit der Betriebsart " Koagulation " nach Anspruch 15 niedrigerer Spannung eingefügt wird, falls die Zeitdauer bis zum Auftreten eines Lichtbogens unter einem voreingestellten Grenzwert liegt.

33. Hochfrequenzgenerator nach einem der Ansprüche 7 bis 32,
**dadurch gekennzeichnet, daß**
anschließend an ein Zeitintervall mit der Betriebsart " "Koagulation" nach Anspruch 15 ein weiteres Zeitintervall mit der Betriebsart "Koagulation" nach Anspruch 16 angefügt wird.

34. Hochfrequenzgenerator nach einem der Ansprüche 7 bis 33,
**dadurch gekennzeichnet, dass** in der Betriebsart" Koagulation " nach Anspruch 16 mit Pulspaketen koaguliert wird, die so lange dauern, dass am Ende der Pulspakete ein Lichtbogen vorgegebener Intensität auftritt.

35. Hochfrequenzgenerator nach Anspruch 7 bis 33,
**dadurch gekennzeichnet, dass** die Spannung des Generators (1) zu Beginn eines Zeitintervalles mit der Betriebsart" Schneiden " bei einer hohen Gewebeimpedanz erhöht wird.

36. Hochfrequenzchirurgiegenerator nach Anspruch 1,
**dadurch gekennzeichnet, dass** eine einstellbaren Ausgangsleistung für miteinander gekoppelte Betriebsarten Koagulieren und nachfolgendes Schneiden zur Entfernung der Sonde vorgesehen ist, und dass eine Steuereinheit (30) vorhanden ist, die nach der Aktivierung des Generators zunächst die erste Betriebsart (a) des Generators mit koagulierender Wirkung einstellt und automatisch am Ende der Koagulation die zweite Betriebsart (b) des Generators mit schneidender Wirkung derart kurzzeitig einstellt, dass nur die gewünschte Loslösung der Sonde vom Gewebe erfolgt, ohne dass die Sonde wesentlich in das Gewebe eindringt.

37. Hochfrequenzgenerator nach Anspruch 36,
**dadurch gekennzeichnet, dass**
in der Steuereinheit (30) ein Zeitgeber (10) vorhanden ist, der am Ende des Zeitintervalles mit der ersten Betriebsart (a) des Generators mit koagulierender Wirkung für das darauffolgende Intervall mit der zweiten Betriebsart (b) des Generators mit schneidender Wirkung eine zu Beginn dieses Intervalles mit der zweiten Betriebsart (b) vorbestimmte Zeit vorgibt, die so klein ist, daß die Sonde (8.1) nicht wesentlich in das Gewebe eindringen kann.

38. Hochfrequenzgenerator nach Anspruch 36 oder 37,
**dadurch gekennzeichnet, daß**
eine Meßeinrichtung (31) mit Auswerteeinrichtung zur Messung der elektrischen Parameter am Generatorausgang während der ersten Betriebsart (a) des Generators mit koagulierender Wirkung vorhanden ist, die aufgrund der gemessenen Signale die optimale Zeitdauer für das folgende Zeitintervall mit der zweiten Betriebsart (b) des Generators mit schneidender Wirkung ermittelt und ein Zeitgeber (10) vorhanden ist, dem das Ausgangssignal der Meßeinrichtung (31) zugeführt ist und den Zeitgeber (10) für das Zeitintervall mit der zweiten Betriebsart (b) des Generators mit schneidender Wirkung geeignet einstellt, daß nur die gewünschte Loslösung der Sonde vom Gewebe erfolgt, ohne daß die Sonde wesentlich in das Gewebe eindringt.

39. Hochfrequenzgenerator nach Anspruch 36,
**dadurch gekennzeichnet, daß**
eine Auswerteschaltung (32) zur mittelbaren und/oder unmittelbaren Feststellung des Schneidens vorhanden ist, die in der zweiten Betriebsart (b) des Generators mit schneidender Wirkung ein Signal an die Steuereinheit (30) abgibt, wenn die Sonde (8.1) zu schneiden beginnt, so daß die Steuereinheit (30) das Zeitintervall mit der zweiten Betriebsart (b) eine voreinstellbare Zeit nach der Feststellung des gewebetrennen den Schneidens derart beendet, daß nur die gewünschte Loslösung der Sonde vom Gewebe erfolgt, ohne daß die Sonde wesentlich in das Gewebe eindringt.

40. Hochfrequenzgenerator nach Anspruch 39,
**dadurch gekennzeichnet, daß**
die Auswerteschaltung (32) zur mittelbaren und/oder unmittelbaren Feststellung des gewebetrennenden Schneidens einen mechanischen Sensor enthält, der ein Eindringen der Sonde (8.1) in das Gewebe feststellt.

41. Hochfrequenzgenerator nach Anspruch 9 und 39,
**dadurch gekennzeichnet, daß**
die Auswerteschaltung (32) zur mittelbaren und/oder unmittelbaren Feststellung des gewebetrennenden Schneidens eine Einrichtung zur Messung der Impedanz des Gewebes enthält und einen Schwellwertdetektor enthält der die beim Beginn des Schneidens typischerweise auftretende Änderung der Gewebeimpedanz erkennt.

42. Hochfrequenzgenerator nach Anspruch 39,
**dadurch gekennzeichnet, daß**
die Auswerteschaltung (32) zur mittelbaren und/oder unmittelbaren Feststellung des gewebetrennenden Schneidens eine Einrichtung zur Messung der spektralen Anteile des Generatorsignals und/oder eines Hilfssignals enthält, die die typischerweise durch den beim Schneiden auftretenden Lichtbogen hervorgerufenen Harmonischen des Generatorsignals und/oder des Hilfssignals detektiert.

## Claims

1. Surgical high-frequency generator (1) for controlled sectioning and coagulation, comprising
- a high-frequency generator issuing an output signal applied to a probe,
- a controller for setting the instantaneous electrical output quantities of the high-frequency generator such as current, voltage or power,
- an electronic set-point generator (9) producing an output signal that is applied as set-point to said controller for setting the development of the electric output quantities of the high-frequency generator versus time in correspondence with the selected mode of operation,
- a measuring means (7) for direct and/or indirect detection of the tissue condition in the vicinity of the electrode of said probe (8.1) and a timer (10),
**characterised by** the following features:
- an electronic memory with an electronic evaluation system is provided for storing the output signal of said measuring means,
- by the end of each interval preset by said timer, said set-point generator for adjusting the set-point for said controller evaluates the output signal from said memory with the electronic evaluation system and the instantaneous output signal from said measuring means.

2. High-frequency generator according to Claim 1,
**characterised in that** a decider (6) is provided which is supplied with the output quantity (5) from said measuring means (7) together with the output quantity (24) from said electronic memory with the electronic evaluation system (23), and which, by the end (15) of each preceding interval (12), is used to take a decision automatically with respect to the selection of the mode of operation for the interval (13) then following.

3. High-frequency generator according to Claim 1 or 2,
**characterised in that** a cut-off element (2) is provided which is used to set the duration of the following interval (13) on the basis of the output quantity of said means (7) for direct and/or indirect detection of the condition of the tissue for the set mode of operation.

4. High-frequency generator according to any of the Claims 1 to 3,
**characterised in that** said means (7) for direct and/or indirect detection of the condition of the tissue in the vicinity of said cutting electrode includes a measuring detector (4) in the immediate vicinity of said cutting electrode (8.1).

5. High-frequency generator according to any of the Claims 1 to 3,
**characterised in that** said means (7) for direct and/or indirect detection of the condition of the tissue comprises a measuring means for detection and an evaluation means (3) for evaluating the electric output quantities of the generator and derives therefrom a suitable signal for the indirect detection of the condition of the tissue.

6. High-frequency generator according to any of the Claims 1 to 3,
**characterised in that** said means (7) for direct and/or indirect detection of the condition of the tissue comprises an electric test signal generator (21) issuing a test signal supplied to said cutting electrode, and that a test signal measuring means (22) is provided for detecting the condition of the tissue in the vicinity of said cutting electrode.

7. High-frequency generator according to any of the Claims 1 to 6,
**characterised in that** fundamentally different modes of operation are provided with different surgical objectives, whereof one "cutting" mode relates to sectioning whilst the other one relates to the "coagulation" mode, and that possibly further modes of operation are provided for selection for different types of sectioning as well as different types of coagulation.

8. High-frequency generator according to Claim 7,
**characterised in that** during one mode of operation the output power of the generator is selected to be negligibly small in the corresponding intervals for surgical reasons.

9. High-frequency generator according to any of the Claims 1 to 8,
**characterised in that**
a means is provided in said measuring means (7) for detecting the tissue impedance present at said cutting electrode (8.1) and for detecting this impedance with reference to said test signals or said electric output quantities of the generator,
and that by the end (15) of the preceding interval (12), the mode of operation is set to coagulation for the following interval (13) when the load impedance is lower than a specified set value, and
that the "cutting" mode is set when the load impedance exceeds this set value.

10. High-frequency generator according to any of the Claims 7 to 9,
**characterised in that** at least four different modes of operation are provided with different surgical objectives, whereof one relates to the "cutting" mode and two others relate to "coagulation" modes.

11. High-frequency generator according to any of the Claims 1 to 10,
**characterised in that** during the "cutting mode" the output voltage of the high-frequency generator (1) is controlled to a predetermined constant value.

12. High-frequency generator according to any of the Claims 1 to 10,
**characterised in that** in the "cutting" mode the output current of the high-frequency generator (1) is controlled to a predetermined constant value.

13. High-frequency generator according to any of the Claims 1 to 10,
**characterised in that** in the "cutting" mode the output power of the high-frequency generator (1) is controlled to a predetermined constant value.

14. High-frequency generator according to any of the Claims 1 to 10,
**characterised in that** in the "cutting" mode the electric arc formed on said cutting electrode (8.1) during sectioning is controlled to a predetermined constant value.

15. High-frequency generator according to any of the Claims 1 to 14,
**characterised in that** in the "coagulation" mode coagulation is optionally performed with a constant voltage or a voltage variable in terms of time, which is so low that an electric arc cannot occur on said cutting electrode (8.1).

16. High-frequency generator according to any of the Claims 1 to 14,
**characterised in that** in the "coagulation" mode coagulation is performed with pulse packages of a voltage that is so high that an electric arc occurs on said cutting electrode (8.1).

17. High-frequency generator according to Claims 10 to 14,
**characterised in that** one of said two different modes of operation corresponds to a coagulation operation according to Claim 15 and the other mode corresponds to a coagulation operation according to Claim 16.

18. High-frequency generator according to Claims 7 to 17,
**characterised in that** in the "cutting" mode the generator (1) is so controlled that the section penetrates into a depth in the tissue, which is greater, by one electrode diameter at maximum than the depth of the preceding coagulation operations.

19. High-frequency generator according to Claim 18,
**characterised in that** in the "cutting" mode the section is terminated when the impedance of the tissue undergoes a strong reduction.

20. High-frequency generator according to any of the Claims 1 to 19,
**characterised in that** the intervals (11) have each a predetermined constant duration and that the instantaneous output quantity of the generator (1) such as voltage, current, power is determined from the preceding period.

21. High-frequency generator according to any of the Claims 1 to 19,
**characterised in that** the output quantity of the generator (1) is set to a value predetermined for each interval, and that the duration of said intervals (11) is determined in each case from the preceding period.

22. High-frequency generator according to any of the Claims 1 to 19,
**characterised in that** the output quantity of the generator (1) is set to a predetermined value that derives from the measurements made in the preceding interval, and that the duration of said intervals (11) is determined in each case from the preceding period.

23. High-frequency generator according to Claim 22,
**characterised in that** the output voltage of the generator is determined from the tissue impedance occurring during the preceding interval.

24. High-frequency generator according to any of the Claims 7 to 23,
**characterised in that** during an interval with the "coagulation" mode coagulation is performed at least at the same depth as sectioning was performed in the last preceding interval with the "cutting" mode.

25. High-frequency generator according to any of the Claims 7 to 24,
**characterised in that** after an interval with the "cutting" mode said first interval with the "coagulation" mode corresponds to a coagulation operation according to Claim 15.

26. High-frequency generator according to any of the Claims 7 to 25,
**characterised in that** at the beginning of an interval with the "coagulation" mode a decision is made on the basis of the tissue impedance whether coagulation according to Claim 15 or coagulation according to Claim 16 is performed, with coagulation according to Claim 16 being selected in the case of a high tissue impedance and with coagulation according to Claim 15 being selected in the case of a low tissue impedance.

27. High-frequency generator according to any of the Claims 7 to 26,
**characterised in that** in the "coagulation" mode coagulation according to Claim 15 is terminated upon occurrence of an electric arc on said cutting electrode (8.1).

28. High-frequency generator according to Claim 27,
**characterised in that** the output voltage of the generator rises in correspondence from a predetermined function, starting out from a predetermined starting value, until an electric arc occurs on said cutting electrode (8.1).

29. High-frequency generator according to Claim 28,
**characterised in that** the rise of the output voltage is determined from the preceding interval in such a way that the time up to occurrence of an electric arc will approximately correspond to a preset value.

30. High-frequency generator according to any of the Claims 7 to 27,
**characterised in that** in the "coagulation" mode the output voltage of the generator is selected on the basis of the occurrence of an electric arc in the preceding interval.

31. High-frequency generator according to any of the Claims 7 to 27,
**characterised in that** in the "coagulation" mode the output voltage of the generator is increased when after a predetermined period an electric arc has not yet occurred.

32. High-frequency generator according to Claim 27,
**characterised in that** an additional interval with a negligibly small generator power and a further interval with the "coagulation" mode according to Claim 15 at lower voltage is inserted if the period up to occurrence of an electric arc is below a preset threshold.

33. High-frequency generator according to any of the Claims 7 to 32,
**characterised in that** subsequently to an interval with the "coagulation" mode according to Claim 15 a further interval with the "coagulation" mode according to Claim 16 is added.

34. High-frequency generator according to any of the Claims 7 to 33,
**characterised in that** in the "coagulation" mode according to Claim 16 coagulation is performed with pulse packages lasting so long that by the ends of said pulse packages an electric arc occurs with a predetermined intensity.

35. High-frequency generator according to Claims 7 to 33,
**characterised in that** the voltage of the generator (1) is increased at the beginning of an interval with the "cutting" mode in the case of high tissue impedance.

36. Surgical high-frequency generator according to Claim 1,
**characterised in that** an adjustable output power is provided for the mutually coupled modes of coagulation and subsequent sectioning for removal of the probe, and that a controller (30) is provided that after activation of the generator initially sets the first mode of operation (a) of the generator for producing a coagulating effect and automatically, by the end of coagulation, the second mode of operation (b) of the generator for a sectioning effect for such a short duration that only the desired detachment of the probe from the tissue will take place without the probe penetrating substantially into the tissue.

37. High-frequency generator according to Claim 36,
**characterised in that** a timer (10) is provided in said controller (30), which, by the end of the interval with said first mode (a) of the generator for producing a coagulating effect, predetermines a period for the following interval with said second mode (b) of the generator for producing a sectioning effect, which period is predetermined by the beginning of this interval with said second mode (b) and which is so small that said probe (8.1) cannot substantially penetrate into the tissue.

38. High-frequency generator according to Claim 36 or 37,
**characterised in that** a measuring means (31) with an evaluation system is provided for measuring the electrical parameters at the generator output during said first mode (a) of the generator for producing a coagulating effect, which means determines, on the basis of the measured signals, the optimum duration for the following interval with said second mode (b) of the generator for producing a sectioning effect, and that a timer (10) is provided which is supplied with the output signal of said measuring means (31) and which sets said timer (10) for said interval with said second mode (b) of the generator for producing a sectioning effect in such a suitable manner that only the desired detachment of the probe from the tissue will take place without a substantial penetration of said probe into the tissue.

39. High-frequency generator according to Claim 36,
**characterised in that** an evaluation circuit (32) is provided for indirect and/or direct detection of sectioning, which, during said second mode (b) of the generator for producing a sectioning effect, issues a signal to said controller (30) when said probe (8.1) begins sectioning, so that said controller (30) terminates the interval with said second mode (b) after a pre-settable period following the detection of the tissue-sectioning cutting operation in such a way that only the desired detachment of said probe from the tissue will take place without a substantial penetration of said probe into the tissue.

40. High-frequency generator according to Claim 39,
**characterised in that** said evaluation circuit (32) comprises a mechanical sensor for indirect and/or direct detection of the tissue-sectioning cutting operation, which sensor detects penetration of said probe (8.1) into the tissue.

41. High-frequency generator according to Claims 9 and 39,
**characterised in that** said evaluation circuit (32) for indirect and/or direct detection of the tissue-sectioning cutting operation comprises a means for measuring the impedance in the tissue as well as a threshold detector that recognises the variation of the tissue impedance that occurs typically when the sectioning operation begins.

42. High-frequency generator according to Claim 39,
**characterised in that** said evaluation circuit (32) for indirect and/or direct detection of the tissue-sectioning cutting operation comprises a means for measuring the spectral fractions of the generator signal and/or an auxiliary signal, which means detects the harmonics of said generator signal and/or said auxiliary signal which are typically induced by the electric arc occurring during a sectioning operation.

## Revendications

1. Générateur haute-fréquence utilisé en chirurgie (1) pour couper et coaguler de manière réglable, comprenant
- un générateur haute-fréquence, qui fournit un signal de sortie appliqué à une sonde,
- une unité de commande à régler les grandeurs de sortie électriques instantanées du générateur haute-fréquence, par exemple le courant, la tension ou la puissance,
- un ajusteur de la valeur de consigne (9) électronique, qui produit un signal de sortie qui est appliqué comme valeur de consigne à ladite unité de commande afin de régler la loi des grandeurs de sortie électriques du générateur haute-fréquence en fonction du temps en correspondance avec le mode d'opération choisi,
- un moyen de mesure (7) pour la détection directe et/ou indirecte de l'état du tissu dans le voisinage de l'électrode de ladite sonde (8.1) et une minuterie (10),
**caractérisé par** les mesures suivantes:
- une mémoire électronique à un moyen analyseur électronique est disposée à mémoriser le signal de sortie dudit moyen de mesure,
- à la fin de chaque intervalle pré-réglé par ladite minuterie, ledit ajusteur de la valeur de consigne à ajuster la valeur de consigne pour ladite unité de commande analyse le signal de sortie de ladite mémoire à moyen analyseur électronique ainsi que le signal de sortie instantané dudit moyen de mesure.

2. Générateur haute-fréquence selon la revendication 1,
**caractérisé en ce qu'**un bloc de décision (6) est disposé, qui est alimenté en la grandeur de sortie (5) dudit moyen de mesure (7) ensemble avec la grandeur de sortie (24) de ladite mémoire électronique à moyen analyseur électronique (23), et qui, à la fin (15) de chaque intervalle précédant (12), est utilisé à prendre une décision, de façon automatisée, en ce qui concerne le choix du mode d'opération pour l'intervalle (13) suivant.

3. Générateur haute-fréquence selon la revendication 1 ou 2,
**caractérisé en ce qu'**un élément coupe-circuit (2) est compris, qui est employé à régler la durée de l'intervalle suivant (13) à la base de la grandeur de sortie dudit moyen (7) pour la détection directe et/ou indirecte de l'état du tissu pour le mode d'opération ajusté.

4. Générateur haute-fréquence selon une quelconque des revendications 1 à 3,
**caractérisé en ce que** ledit moyen (7) de détection directe et/ou indirecte de l'état du tissu dans le voisinage de ladite électrode tranchante comprend un détecteur mesureur (4) à proximité directe de ladite électrode tranchante (8.1).

5. Générateur haute-fréquence selon une quelconque des revendications 1 à 3,
**caractérisé en ce que** ledit moyen (7) à détection directe et/ou indirecte de l'état du tissu comprend un moyen de mesure à détecter et un moyen analyseur (3) à analyser les grandeurs de sortie électriques du générateur, en dérivant un signal approprié pour la détection indirecte de l'état du tissu.

6. Générateur haute-fréquence selon une quelconque des revendications 1 à 3,
**caractérisé en ce que** ledit moyen (7) de détection directe et/ou indirecte de l'état du tissu comprend un générateur électrique du signal de contrôle (21), qui fournit un signal de contrôle alimenté à ladite électrode tranchante, et **en ce qu'**un moyen mesureur du signal de contrôle (22) est disposé à détecter l'état du tissu dans le voisinage de ladite électrode tranchante.

7. Générateur haute-fréquence selon une quelconque des revendications 1 à 6,
**caractérisé en ce que** des modes d'opération fondamentalement différents sont prévus dans les buts chirurgicaux différents, dont l'un est un mode de "sectionnement", qui se réfère au sectionnement, pendant que l'autre se réfère à un mode de "coagulation", et **en ce que** des autres modes d'opérations éventuels sont prévus au choix pour des types différents de sectionnement ainsi que pour des types différents de coagulation.

8. Générateur haute-fréquence selon la revendication 7,
**caractérisé en ce qu'**au cours d'un mode d'opération la puissance de sortie du générateur est choisie à un niveau négligeable dans les intervalles correspondants pour des raisons chirurgicales.

9. Générateur haute-fréquence selon une quelconque des revendications 1 à 8,
**caractérisé en ce que**
un moyen est disposé dans ledit moyen de mesure (7) à détecter l'impédance dans le tissu, qui est présente à ladite électrode tranchante (8.1), et à détecter cette impédance avec référence auxdits signaux de contrôles ou lesdites grandeurs de sortie électriques du générateur,
et **en ce qu'**à la fin (15) de l'intervalle précédant (12), le mode d'opération est ajusté à coagulation pour l'intervalle suivant (13) quand l'impédance de charge est plus petite qu'une valeur de consigne spécifiée, et
**en ce que** le mode de "sectionnement" est ajusté quand l'impédance de charge dépasse cette valeur de consigne.

10. Générateur haute-fréquence selon une quelconque des revendications 7 à 9, **caractérisé en ce qu'**au moins quatre modes d'opération différents sont prévus dans des buts chirurgicaux différents, dont l'un se réfère au mode de "sectionnement" et les deux autres se réfère aux modes de "coagulation" modes.

11. Générateur haute-fréquence selon une quelconque des revendications 1 à 10, **caractérisé en ce que** pendant le "mode sectionnant" la tension de sortie du générateur haute-fréquence (1) est réglée à une valeur constante déterminée.

12. Générateur haute-fréquence selon une quelconque des revendications 1 à 10, **caractérisé en ce qu'**au mode de "sectionnement" le courant de sortie du générateur haute-fréquence (1) est réglé à une valeur constante déterminée.

13. Générateur haute-fréquence selon une quelconque des revendications 1 à 10, **caractérisé en ce qu'**au mode de "sectionnement" la puissance de sortie du générateur haute-fréquence (1) est réglée à une valeur constante déterminée.

14. Générateur haute-fréquence selon une quelconque des revendications 1 à 10, **caractérisé en ce qu'**au mode de "sectionnement" l'arc électrique établi sur ladite électrode tranchante (8.1) au cours du sectionnement est réglé à une valeur constante déterminée.

15. Générateur haute-fréquence selon une quelconque des revendications 1 à 14, **caractérisé en ce qu'**au mode de "coagulation" la coagulation est réalisée à volonté à une tension constante ou à une tension variable en fonction du temps, qui est si basse qu'un arc électrique ne puisse être établi sur ladite électrode tranchante (8.1).

16. Générateur haute-fréquence selon une quelconque des revendications 1 à 14, **caractérisé en ce qu'**au mode de "coagulation" la coagulation est réalisée aux paquets d'impulsions d'une tension qui est si haute qu'un arc électrique puisse s'établir sur ladite électrode tranchante (8.1).

17. Générateur haute-fréquence selon les revendications 10 à 14,
**caractérisé en ce qu'**un desdits deux modes d'opération différents correspond à une opération de coagulation selon la revendication 15 et l'autre mode correspond à une opération de coagulation selon la revendication 16.

18. Générateur haute-fréquence selon les revendications 7 à 17,
**caractérisé en ce qu'**au mode de "sectionnement" le générateur (1) est réglé de façon que le sectionnement pénètre dans une profondeur dans le tissu, qui est plus grande, par un diamètre d'électrode au maximum, que la profondeur des opérations de coagulation précédentes.

19. Générateur haute-fréquence selon la revendication 18,
**caractérisé en ce qu'**au mode de "sectionnement" le sectionnement est terminé dès que l'impédance du tissu subit une forte réduction.

20. Générateur haute-fréquence selon une quelconque des revendications 1 à 19, **caractérisé en ce que** chacun des intervalles (11) est d'une durée constante déterminée, et **en ce que** la grandeur de sortie du générateur (1) instantanée, par exemple la tension, le courant, la puissance, est déterminée à la base de la période précédente.

21. Générateur haute-fréquence selon une quelconque des revendications 1 à 19, **caractérisé en ce que** la grandeur de sortie du générateur (1) est ajustée à une valeur déterminée pour chaque intervalle, et **en ce que** la durée desdits intervalles (11) est déterminée en chaque cas à la base de la période précédente.

22. Générateur haute-fréquence selon une quelconque des revendications 1 à 19, **caractérisé en ce que** la grandeur de sortie du générateur (1) est ajustée à une valeur déterminée, qui est dérivée de mesures faites dans l'intervalle précédant, et **en ce que** la durée desdits intervalles (11) est déterminée en chaque cas à la base de la période précédente.

23. Générateur haute-fréquence selon la revendication 22,
**caractérisé en ce que** la tension de sortie du générateur est déterminée en la dérivant de l'impédance dans le tissu, qui est présente au cours de l'intervalle précédent.

24. Générateur haute-fréquence selon une quelconque des revendications 7 à 23, **caractérisé en ce qu'**au cours d'un intervalle au mode de "coagulation", la coagulation se fait au moins à la même profondeur que la profondeur de sectionnement au cours du dernier intervalle précédant au mode de "sectionnement".

25. Générateur haute-fréquence selon une quelconque des revendications 7 à 24, **caractérisé en ce qu'**après un intervalle au mode de "sectionnement", ledit premier intervalle au mode de "coagulation" correspond à une opération de coagulation selon la revendication 15.

26. Générateur haute-fréquence selon une quelconque des revendications 7 à 25, **caractérisé en ce qu'**au début d'un intervalle au mode de "coagulation", une décision est prise à la base de l'impédance dans le tissu, si la coagulation selon la revendication 15 ou la coagulation selon la revendication 16 est réalisée, à la coagulation selon la revendication 16 étant choisie au cas d'une haute impédance dans le tissu et à la coagulation selon la revendication 15 étant choisie au cas d'une basse impédance dans le tissu.

27. Générateur haute-fréquence selon une quelconque des revendications 7 à 26, **caractérisé en ce qu'**au mode de "coagulation", la coagulation selon la revendication 15 est terminée dès qu'un arc électrique est établi sur ladite électrode tranchante (8.1).

28. Générateur haute-fréquence selon la revendication 27,
**caractérisé en ce que** la tension de sortie du générateur monte en correspondance d'une fonction déterminée, en départ d'une valeur de début déterminée jusqu'à l'établissement d'un arc électrique sur ladite électrode tranchante (8.1).

29. Générateur haute-fréquence selon la revendication 28,
**caractérisé en ce que** la montée de la tension de sortie est déterminée par dérivation de l'intervalle précédant de façon que le temps jusqu'à l'établissement d'un arc électrique corresponde approximativement à une valeur pré-réglée.

30. Générateur haute-fréquence selon une quelconque des revendications 7 à 27, **caractérisé en ce qu'**au mode de "coagulation" la tension de sortie du générateur est choisie à la base de l'établissement d'un arc électrique au cours de l'intervalle précédent.

31. Générateur haute-fréquence selon une quelconque des revendications 7 à 27, **caractérisé en ce qu'**au mode de "coagulation" la tension de sortie du générateur est augmentée quand, après une période déterminée, il n'y avait pas encore un établissement d'un arc électrique.

32. Générateur haute-fréquence selon la revendication 27,
**caractérisé en ce qu'**un intervalle supplémentaire, à puissance de générateur négligeable, et un autre intervalle au mode de "coagulation" selon la revendication 15 à plus basse tension sont insérés si la période jusqu'à l'établissement d'un arc est plus petite qu'un seuil déterminé.

33. Générateur haute-fréquence selon une quelconque des revendications 7 à 32, **caractérisé en ce que** suivant un intervalle au mode de "coagulation" selon la revendication 15, un autre intervalle au mode de "coagulation" selon la revendication 16 est inclus.

34. Générateur haute-fréquence selon une quelconque des revendications 7 à 33, **caractérisé en ce qu'**au mode de "coagulation" selon la revendication 16 la coagulation se fait aux paquets d'impulsions d'une durée si longue qu'aux fins desdits paquets d'impulsions un arc électrique soit établi à une intensité déterminée.

35. Générateur haute-fréquence selon les revendications 7 à 33,
**caractérisé en ce que** la tension du générateur (1) est augmenté au début d'un intervalle au mode de "sectionnement" au cas d'une haute impédance dans le tissu.

36. Générateur haute-fréquence utilisé en chirurgie selon la revendication 1,
**caractérisé en ce qu'**une puissance de sortie réglable est prévue pour les modes de coagulation combinés l'un à l'autre et de sectionnement ultérieur pour le retrait de la sonde, et **en ce qu'**une unité de commande (30) est disposée, qui, après la mise en circuit du générateur, commute d'abord au premier mode d'opération (a) du générateur afin de produire un effet de coagulation, et, de façon automatisée, à la fin de la coagulation, au deuxième mode d'opération (b) du générateur afin de créer un effet de sectionnement pour une durée si courte que seulement le détachement souhaité de la sonde du tissu soit achevé, sans pénétration essentielle de la sonde dans le tissu.

37. Générateur haute-fréquence selon la revendication 36,
**caractérisé en ce qu'**une minuterie (10) est disposé dans ladite unité de commande (30), qui, à la fin de l'intervalle audit premier mode (a) du générateur à produire un effet de coagulation, détermine une période pour l'intervalle suivant audit deuxième mode (b) du générateur à produire un effet de sectionnement, laquelle période est déterminée au début de cet intervalle audit deuxième mode (b) et si petit que ladite sonde (8.1) ne puisse pénétrer essentiellement dans le tissu.

38. Générateur haute-fréquence selon la revendication 36 ou 37,
**caractérisé en ce qu'**un moyen de mesure (31) à moyen analyseur est disposé à mesurer les paramètres électriques à la sortie du générateur pendant ledit premier mode (a) du générateur afin de produire un effet de coagulation, ce moyen déterminant, à la base des signaux mesurés, la durée optimale pour l'intervalle suivant audit deuxième mode (b) du générateur afin de produire un effet de sectionnement, et **en ce qu'**une minuterie (10) est comprise, à laquelle est fourni le signal de sortie dudit moyen de mesure (31) qui règle ladite minuterie (10) pour ledit intervalle audit deuxième mode (b) du générateur afin de produire un effet de sectionnement d'une manière convenable, que seulement de détachement souhaité de la sonde du tissu soit achevé sans pénétration essentielle de ladite sonde dans le tissu.

39. Générateur haute-fréquence selon la revendication 36,
**caractérisé en ce qu'**un circuit analyseur (32) est compris pour la détection indirecte et/ou directe du sectionnement, qui, au cours dudit deuxième mode (b) du générateur afin de produire un effet de sectionnement, fournit un signal à ladite unité de commande (30) dès que ladite sonde (8.1) commence le sectionnement, de façon que ladite unité de commande (30) termine l'intervalle audit deuxième mode (b) après une période pré-réglable suivant la détection de l'opération de sectionnement à couper le tissu, d'une manière que seulement de détachement souhaité de la sonde du tissu soit achevé sans pénétration essentielle de ladite sonde dans le tissu.

40. Générateur haute-fréquence selon la revendication 39,
**caractérisé en ce que** ledit circuit analyseur (32) comprend un détecteur mécanique pour la détection indirecte et/ou directe de l'opération de sectionnement, ce détecteur détectant la pénétration de ladite sonde (8.1) dans le tissu.

41. Générateur haute-fréquence selon les revendications 9 et 39,
**caractérisé en ce que** ledit circuit analyseur (32) pour la détection indirecte et/ou directe de l'opération de sectionnement à couper le tissu, comprend un moyen à mesurer l'impédance dans le tissu ainsi qu'un détecteur de seuil, qui détecte la variation de l'impédance dans le tissu, qui est présente, au cas typique, au début de l'opération de sectionnement.

42. Générateur haute-fréquence selon la revendication 39,
**caractérisé en ce que** ledit circuit analyseur (32) pour la détection indirecte et/ou directe de l'opération de sectionnement à couper le tissu comprend un moyen à mesurer les fractions spectrales du signal du générateur et/ou d'un signal auxiliaire, ce moyen détectant des harmoniques dudit signal du générateur et/ou dudit signal auxiliaire, qui sont induits, au cas typique, par l'arc électrique établi au cours d'une opération de sectionnement.
